# FASCICULE DE BREVET EUROPEEN

(11) **EP 1 664 723 B1**
(45) Date de publication et mention de la délivrance du brevet: **10.10.2018**
(21) Numéro de dépôt: 04786278.4
(22) Date de dépôt: 06.08.2004
(51) Int. Cl.: B01F 9/00, G01N 33/49, G01N 35/10, B01F 11/00, G01N 35/00

(54) **ANALYSEUR HÉMATOLOGIQUE SUR SANG TOTAL AVEC DISPOSITIF D'AGITATION**
HÄMATOLOGISCHES ANALYSEGERÄT FÜR VOLLBLUT MIT RÜHRVORRICHTUNG
HEMATOLOGICAL ANALYZER ON WHOLE BLOOD WITH STIRRING DEVICE

(30) Priorité: 26.08.2003 FR 0310175
(43) Date de publication de la demande: 07.06.2006
(73) Titulaire: HORIBA ABX SAS, 34090 Montpellier (FR)
(72) Inventeur: DUPOTEAU, François, F-75018 Paris (FR); LE COMTE, Roger, F-34470 Perols (FR); COUDERC, Guilhem, F-34430 Saint Jean de Vedas (FR)
(74) Mandataire: Plaçais, Jean Yves
(86) Numéro de dépôt international: PCT/FR2004/002105
(87) Numéro de publication internationale: WO 2005/022168

(56) Documents cités:
- EP-A- 1 174 717
- US-A- 4 518 264
- US-A- 4 921 676
- US-A- 5 110 743

## Description

L'invention se rapporte au domaine des analyseurs hématologiques destinés à analyser automatiquement des échantillons de produits sanguins.

Plus précisément, elle concerne un analyseur hématologique sur sang total, pour l'analyse de sangs contenus dans des tubes, dans lequel les tubes sont amenés et traités dans un mode tube par tube.

Dans ce qui suit, on entend par "fonctionnement en mode unitaire" ou "fonctionnement en mode tube par tube", un mode de fonctionnement dans lequel les échantillons de sang à analyser sont passés l'un après l'autre, c'est-à-dire tube par tube, sans regroupement de ces tubes dans des supports du type cassette ou analogue.

Par l'expression "analyseur sur sang total", on entend désigner un analyseur effectuant des analyses sur du sang total, c'est-à-dire contenant tous les éléments du sang, en opposition aux analyseurs fonctionnant sur plasma ou sérum. Les compteurs de globules font partie des analyseurs sur sang total, mais l'invention ne se limite pas aux seuls compteurs de globules.

Par l'expression "contrôle de qualité", on entend désigner une procédure qui consiste à vérifier, au minimum quotidiennement, le bon fonctionnement de l'analyseur avant d'effectuer des analyses des échantillons de sang des patients.

Il existe différents types d'analyseurs hématologiques qui procèdent à différents types d'analyses, optiques, physico-chimiques, etc. sur des échantillons de sang et qui délivrent ensuite automatiquement les résultats d'analyse.

Une condition essentielle pour le bon fonctionnement de ces analyseurs est que les tubes de sang aient été préalablement correctement agités lors d'une phase d'agitation qui précède l'analyse.

Or, dans les analyseurs hématologiques fonctionnant en mode unitaire ou mode tube par tube, cette phase d'agitation est souvent réalisée manuellement, donc de façon plus ou moins empirique.

Il en résulte que lorsque l'utilisateur de l'analyseur hématologique n'est pas rodé aux pratiques du laboratoire, cette phase préalable d'agitation peut être menée de façon incorrecte, ce qui peut perturber les résultats d'analyse.

Ceci est souvent le cas lorsque les utilisateurs ne sont pas spécifiquement formés aux pratiques du laboratoire.

L'invention a notamment pour but de surmonter ces inconvénients en proposant d'intégrer cette phase d'agitation du sang dans un analyseur hématologique sur sang total.

Il existe plusieurs catégories d'analyseurs fonctionnant à partir de sang total. Une des catégories les plus importantes est celle des compteurs de cellules, mais l'invention ne se limite pas à cette catégorie particulière.

On connaît déjà des analyseurs hématologiques qui fonctionnent, non pas en mode tube par tube, mais dans un mode de regroupement des tubes. Dans ce cas, l'opérateur regroupe le maximum de tubes avant de commencer ses analyses, ce qui lui permet de mieux gérer son temps et de sécuriser les tâches d'identification et de validation des résultats.

Dans ce cas, les tubes sont chargés dans des cassettes ou des plateaux avant le démarrage d'un cycle automatique d'analyse. C'est donc l'analyseur hématologique qui prend en charge l'agitation du sang avant analyse.

En revanche, dans les analyseurs hématologiques fonctionnant en mode unitaire, la phase d'agitation n'est pas réalisée dans l'analyseur lui-même. Elle est généralement effectuée, soit de façon manuelle, soit dans un agitateur séparé ou agitateur externe, fonctionnant par exemple par rotation ou par balancement. L'opérateur doit attendre quelques minutes pour obtenir une agitation parfaite avant de commencer la série d'analyses. Comme les tubes sont retirés de l'agitateur juste avant l'analyse, certains tubes peuvent y séjourner plus longtemps que d'autres.

Par ailleurs, certains laboratoires utilisent le temps compris entre deux analyses pour agiter manuellement le tube suivant.

Certains analyseurs fonctionnent en mode "tube ouvert", c'est-à-dire que l'opérateur prend le tube agité et doit ôter le bouchon avant de présenter le tube à l'analyseur.

D'autres analyseurs fonctionnent en mode "tube fermé", c'est-à-dire que le tube agité est directement présenté à l'analyseur sans ouverture préalable. L'analyseur prend en charge le percement du bouchon afin de prélever le sang à l'intérieur du tube. Ce mode de fonctionnement permet de préserver l'utilisateur de tout risque de contact avec le sang.

Dans tous les cas, une agitation préalable est nécessaire, même si le sang vient juste d'être prélevé sur un patient.

Les analyseurs fonctionnant en mode unitaire sont principalement destinés aux petits laboratoires effectuant peu d'analyses et utilisés conjointement avec un agitateur externe.

En dehors du cadre du laboratoire, il existe une multitude de cas où il est nécessaire d'effectuer des "analyses auprès du patient". Cela peut répondre à un besoin d'obtenir rapidement un résultat, comme dans le cas des unités d'urgence.

Cela peut répondre aussi à un besoin de dépistage, comme cela est pratiqué couramment dans certains pays, tels les États-Unis ou le Japon, où les médecins sont habilités à effectuer des analyses de base leur permettant de consolider leur diagnostic.

Cela peut aussi répondre aux besoins des centres de médecine déportés où il n'existe pas de laboratoires à proximité, comme par exemple, dans les situations de médecine dans des endroits ruraux, de médecine ambulatoire ou de médecine militaire.

Dans toutes ces situations "d'analyses auprès du patient", les opérateurs sont plutôt des médecins ou des infirmiers que des opérateurs de laboratoire qualifiés.

Comme ces analyses sont effectuées sur une très petite quantité de sang, il est nécessaire que cet échantillonnage soit représentatif du sang du patient et donc que le contenu du tube soit parfaitement homogène.

En dehors du contexte du laboratoire, cette phase d'agitation préalable est parfois négligée par méconnaissance de son importance, mais le plus souvent parce qu'elle ne correspond pas aux gestes naturels effectués en conditions d'urgence.

Pour des questions pratiques d'organisation, il n'est pas facile pour un opérateur de poser un tube de sang sur un agitateur, puis de venir ensuite le reprendre pour effectuer l'analyse, comme le ferait un laboratoire après regroupement des tubes à analyser.

Il existe donc un besoin d'automatiser l'agitation dans un analyseur hématologique sur sang total, fonctionnant en mode unitaire ou mode tube par tube.

Dans le domaine des analyseurs hématologiques fonctionnant par regroupement de tubes dans des supports du type cassette ou analogue, il existe déjà des moyens réalisant une agitation automatique.

Ainsi, dans le domaine des analyseurs sur sang total à supports multiples regroupant des tubes de sang, il existe déjà des moyens d'agitation. Le brevet US 5 232 081 décrit notamment un dispositif d'agitation, dans lequel le mixage des échantillons de sang se fait sur un support du type cassette regroupant une multiplicité de tubes. Toutefois, ce dispositif d'agitation n'est valable que pour les appareils à supports multiples.

Il existe déjà différents moyens d'agitation connus, comme par exemple ceux indiqués dans la liste non limitative suivante :
- agitation par retournement complet : ce moyen consiste à renverser verticalement le tube de sang plusieurs fois consécutivement. Le mélange s'effectue pendant le retournement lorsque le sang passe du bas du tube vers le haut du tube et inversement lors du retour à la position de départ. Un exemple d'application est décrit dans le brevet US 5 110 743 ;
- agitation par retournement partiel : dans ce cas, le tube n'effectue pas une rotation totale. Le principe de mélange est le même que celui décrit ci-dessus ;
- agitation par balancement : dans ce cas, le tube est posé sur un plateau horizontal qui effectue un mouvement de balance. Un exemple d'application est décrit dans le brevet US 4 518 264 ;
- agitation par rotation du tube sur lui-même, dite par effet "Vortex" : dans ce type d'agitation, le tube reste en position verticale. Il est entraîné en rotation sur son axe vertical en sens alterné.

L'invention propose un analyseur hématologique sur sang total, fonctionnant en mode tube par tube, qui vise à éviter les inconvénients précités des analyseurs en mode tube par tube.

A cet effet, l'analyseur de l'invention comprend :
- un dispositif d'agitation agencé pour recevoir un tube de sang et agiter ce tube de sang selon un mode d'agitation choisi ;
- des moyens de commande reliés au dispositif d'agitation pour agiter le tube de sang dans des conditions contrôlées selon des paramètres définis ; et
- des moyens de prélèvement agencés pour prélever un échantillon de sang dans le tube de sang préalablement agité par le dispositif d'agitation et transférer l'échantillon de sang vers un bloc d'analyse.

Ainsi, l'invention propose un analyseur hématologique intégrant un dispositif d'agitation, ce qui permet d'automatiser l'agitation, nécessaire avant l'analyse, afin d'en garantir la qualité, y compris sur les sangs destinés au contrôle de qualité.

Un tel analyseur hématologique trouve un intérêt tout particulier dans le cas des appareils de routine de laboratoire, et dans les appareils unitaires utilisés par des opérateurs effectuant des "analyses auprès du patient".

Mais l'invention trouve aussi un intérêt dans les appareils utilisés dans les laboratoires, car elle permet d'unifier les types d'agitation.

Dans tous les cas, l'analyseur hématologique de l'invention permet d'effectuer l'agitation au sein même de l'analyseur, et non pas de manière séparée comme dans la technique antérieure, et cela dans des conditions contrôlées garantissant la qualité de l'agitation.

Dans l'invention, le tube de sang peut être disposé manuellement dans le dispositif d'agitation. Toutefois, dans une version plus évoluée, l'analyseur hématologique peut comporter des moyens de déplacement agencés pour transférer un tube de sang vers le dispositif d'agitation.

Selon une autre caractéristique de l'invention, le dispositif d'agitation comprend un organe de réception pour recevoir un tube de sang soit directement, soit par l'intermédiaire d'un support.

Cet organe de réception peut comporter soit une seule empreinte adaptée à un tube de sang d'un modèle défini, soit plusieurs empreintes adaptées respectivement à des tubes de sang de modèles différents. Cependant, dans tous les cas, l'agitation est réalisée sur un seul tube de sang.

Les moyens de commande de l'analyseur de l'invention comprennent avantageusement des moyens de programmation pour programmer la durée de l'agitation du dispositif d'agitation. Ceci permet de réaliser l'agitation dans des conditions bien maîtrisées.

Le dispositif d'agitation intégré à l'analyseur de l'invention n'est pas limité à un mode d'agitation particulier.

Toutefois, dans une forme préférée de l'invention, le dispositif d'agitation comprend des moyens d'agitation par une succession de retournements complets du tube de sang, ou encore des moyens d'agitation par une succession de retournements partiels du tube de sang.

Il est également possible d'utiliser des moyens d'agitation fonctionnant par une succession de balancements du tube de sang dans un plan vertical.

Dans une autre variante, le dispositif d'agitation comprend des moyens d'agitation par une séquence comprenant la rotation du tube de sang selon un axe vertical avec un sens de rotation donné, un arrêt de l'agitation, une rotation du tube de sang selon cet axe vertical en sens inverse, et un nouvel arrêt de l'agitation.

Il est avantageux que les moyens de commande établissent une durée d'agitation. C'est cette durée d'agitation qui détermine le nombre de retournements complets du tube de sang ou le nombre de retournements partiels du tube de sang.

C'est également cette durée d'agitation qui détermine soit le nombre de balancements du tube de sang, soit le nombre de séquences de rotations et d'arrêts de rotation dans le cas des variantes précitées.

Selon une autre caractéristique de l'invention, les paramètres d'agitation des moyens de commande sont paramétrables soit directement sur l'analyseur hématologique, soit par l'intermédiaire d'une station de contrôle indépendante.

Les paramètres d'agitation sont avantageusement regroupés en profils d'utilisation.

Selon encore une autre caractéristique de l'invention, le dispositif d'agitation et le bloc d'analyse sont agencés pour fonctionner de manière simultanée, ce qui permet d'agiter un tube de sang par les moyens d'agitation, tandis qu'un tube précédemment agité est analysé par le bloc d'analyse.

Les moyens de commande précités peuvent aussi établir une durée d'agitation qui est nulle, ce qui permet de ne pas effectuer une agitation.

Dans l'invention, le tube de sang est habituellement fermé par un bouchon.

Il est avantageux en ce cas de prévoir un capteur optique placé à l'intérieur de l'analyseur hématologique pour détecter la présence ou l'absence d'un bouchon sur le tube de sang.

Les moyens de prélèvement comprennent avantageusement un organe de perçage du bouchon. Celui-ci est de préférence situé en surplomb du tube à percer, c'est-à-dire au-dessus de celui-ci.

Toutefois, il est envisageable aussi de prévoir que l'organe de perçage du bouchon soit situé en dessous du tube à percer.

Selon encore une autre caractéristique de l'invention, l'analyseur hématologique comprend des moyens de connexion interne ou externe permettant l'échange de données sécurisées.

Dans la description qui suit, faite à titre d'exemple, on se réfère aux dessins annexés, sur lesquels :
- la figure 1 est une vue de côté d'un analyseur hématologique avec dispositif d'agitation intégré, conforme à l'invention ;
- la figure 2 est une vue en perspective de l'analyseur hématologique de la figure 1 ;
- la figure 3 montre un dispositif de déplacement propre à introduire automatiquement un tube de sang dans le dispositif d'agitation ;
- la figure 4 est une vue frontale d'un tambour d'agitation comprenant une seule empreinte de tube ;
- la figure 5 est une vue analogue à la figure 4 dans une variante de réalisation comportant plusieurs empreintes aptes à recevoir des tubes de types différents ;
- la figure 6 est une vue de côté d'un tambour d'agitation dans lequel le perçage du tube s'effectue par dessous ;
- la figure 7 illustre le principe d'un agitateur par retournement partiel des tubes ;
- la figure 8 montre un dispositif d'agitation fonctionnant par balancement ;
- la figure 9 montre un dispositif d'agitation fonctionnant par rotation autour d'un axe vertical ; et
- la figure 10 est un schéma illustrant, sous la forme d'un organigramme, le fonctionnement d'un analyseur hématologique selon l'invention.

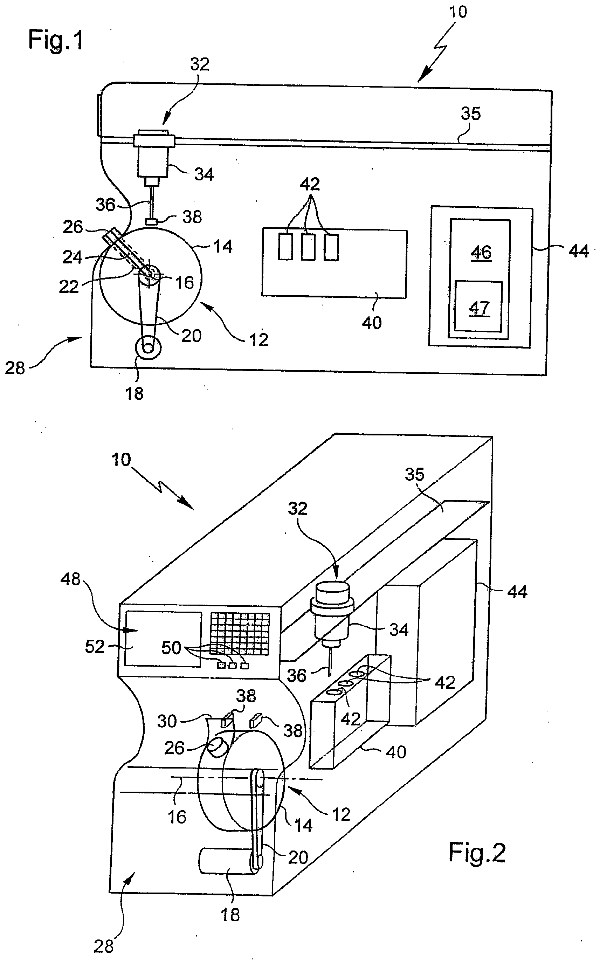

On se réfère d'abord conjointement aux figures 1 et 2 qui représentent un analyseur hématologique désigné dans son ensemble par la référence 10.

Dans l'exemple, il s'agit d'un analyseur destiné à compter les globules de sang, s'agissant de l'une des applications principales des analyses sur sang total. Cependant, comme déjà indiqué, l'invention trouve son application dans tous les analyseurs à caractère unitaire nécessitant du sang total préalablement agité avant analyse.

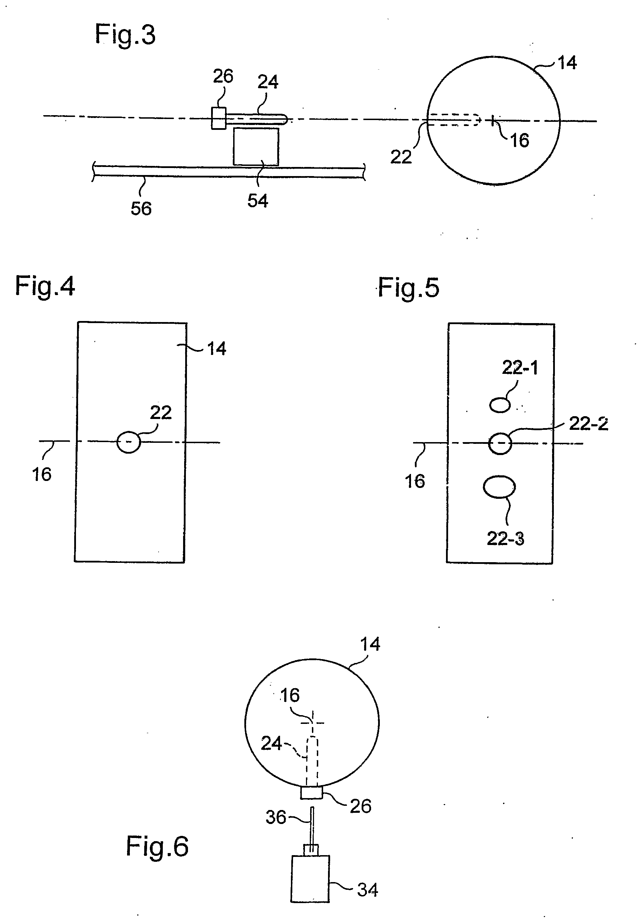

L'analyseur 10 intègre un dispositif d'agitation 12 qui comprend un tambour 14 monté en rotation autour d'un axe horizontal 16 et susceptible d'être entraîné en rotation, dans un sens ou dans l'autre, par un moteur électrique 18, du type pas à pas et d'une courroie de transmission 20. Le tambour 14 comporte une empreinte 22 dirigée radialement (voir aussi la figure 4) qui permet de recevoir un tube de sang 24 fermé par un bouchon 26.

Le dispositif d'agitation 12 est situé près d'une face frontale 28 de l'appareil dans laquelle est aménagée une fenêtre 30 (figure 2) permettant l'introduction du tube 24 dans l'empreinte 22, le tambour 14 étant dans une position angulaire définie. Dans l'exemple, l'empreinte fait un angle d'environ 45° par rapport à l'horizontale, ce qui permet l'introduction du tube dans l'empreinte. Dans l'exemple, cette introduction s'effectue de façon manuelle, mais elle peut aussi être effectuée de façon automatique, comme on le verra plus loin.

L'analyseur 10 comprend en outre des moyens de prélèvement désignés dans leur ensemble par la référence numérique 32. Ils comprennent un bloc de perçage 34 déplaçable le long d'un rail horizontal 35. Le bloc de perçage comprend une aiguille de prélèvement 36 disposée verticalement, avec sa pointe orientée vers le bas. Le bloc de perçage est agencé pour commander le déplacement de l'aiguille verticalement, soit en direction descendante pour venir percer le bouchon 26 d'un tube, soit de manière ascendante une fois le prélèvement effectué. Le prélèvement s'effectue dans une position où le tube 24 est dirigé verticalement, avec son bouchon orienté vers le haut.

La présence du bouchon 26 est détectée par un capteur 38 qui, dans l'exemple, est de type optique.

Lorsque la présence d'un tube est détectée, le bloc de perçage 34 peut entrer en fonctionnement pour percer le bouchon, et prélever une quantité donnée de sang dans le tube. Le bloc de perçage 34 est déplaçable le long du rail pour venir ensuite au-dessus d'un bloc d'analyse 40 comportant des récipients de mesure 42, non décrits en détail, s'agissant de moyens en eux-mêmes connus.

L'analyseur 10 comprend en outre un module d'automatisme 44 qui intègre une unité de commande 46 pour piloter le fonctionnement de l'analyseur 10, comme on le verra plus loin. Cette unité de commande 46 peut comprendre, dans un mode de réalisation, une unité de programmation 47 qui permet de programmer le fonctionnement de l'analyseur 10 et en particulier de régler la durée de l'agitation.

Ce module d'automatisme est commandé à partir d'une interface 48 située en partie supérieure de la face frontale 28 et comprenant des touches 50 et un écran de contrôle 52.

On se réfère maintenant à la figure 3 qui montre une variante de réalisation de l'invention, dans laquelle le tube 24, fermé par son bouchon 26, est introduit dans l'empreinte 22 du tambour 14 par des moyens de déplacement. Ces derniers comprennent un chariot 54 déplaçable en va et vient le long d'un rail horizontal 56. Dans l'exemple, le tube est déplacé avec son axe placé horizontalement et est introduit dans l'empreinte 22, dont l'axe est également disposé horizontalement.

Bien entendu, d'autres types de moyens de déplacement fonctionnant de manière automatique sont utilisables pour introduire le tube automatiquement dans l'empreinte 22 du tambour 14.

Dans l'invention, le tube de sang peut être introduit directement dans le logement de réception (empreinte 22) du tambour 14 ou bien indirectement par l'intermédiaire d'un support individuel dans lequel le tube de sang est placé au départ.

Le tambour 14 de la figure 4 comporte une seule empreinte 22 qui est adaptée à un tube de sang d'un type donné (longueur et diamètre).

Cependant, il existe différents types de tubes qui diffèrent notamment par leurs dimensions (diamètre et longueur).

La forme de réalisation de la figure 5 montre un tambour 14 pouvant recevoir différents types de tubes. A cet effet, le tambour comporte à sa périphérie plusieurs empreintes 22-1, 22-2 et 22-3 susceptibles de s'adapter respectivement à trois types de tubes différents. Cependant, dans tous les cas, l'agitation se fera sur un seul tube préalablement introduit dans l'empreinte qui lui convient.

La figure 6, à laquelle on se réfère maintenant, montre une autre variante de réalisation de l'analyseur 10, dans lequel le bloc de perçage 34 est placé en dessous du tambour 14, l'aiguille de prélèvement 36 étant disposée verticalement avec sa pointe dirigée vers le haut. Ceci permet d'effectuer le perçage du bouchon 26 du tube 24 dans une position où ce bouchon est dirigé vers le bas.

Cependant, il entre également dans le cadre de l'invention de réaliser le perçage du bouchon dans toute position angulaire définie du tube.

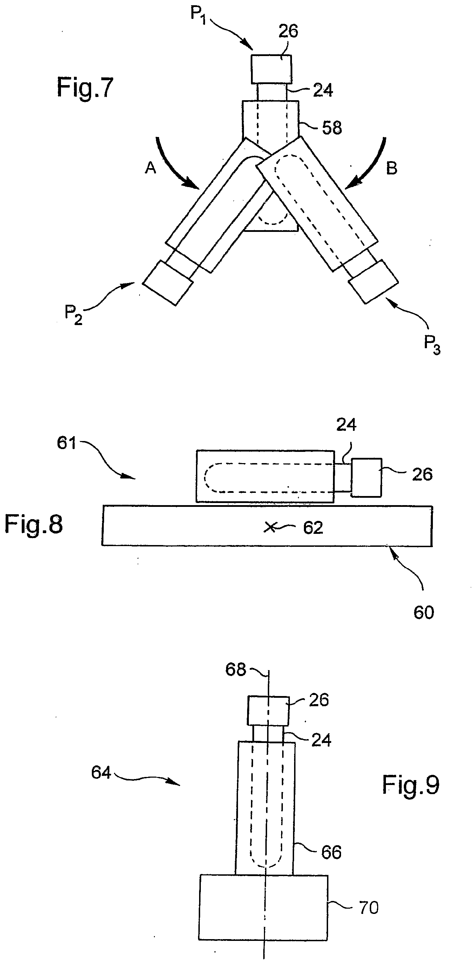

Dans l'exemple de réalisation des figures 1 et 2, le dispositif d'agitation 12 réalise une agitation par retournement dont le principe est illustré à la figure 7.

Dans l'exemple, le tube 24 avec son bouchon 26 est introduit dans un support individuel 58 qui peut être lui-même ensuite introduit dans le tambour 14 (non représenté sur la figure 7). P1 désigne la position de référence (position verticale) avec le bouchon dirigé vers le haut. Dans l'exemple, il s'agit d'une agitation par retournement partiel qui peut se faire par un angle négatif A jusqu'à une position P2 ou par un angle positif B jusqu'à une position P3. L'amplitude du mouvement doit être au minimum de 100° jusqu'à un angle de 180° ou au minimum de - 100° jusqu'à un angle de -180°. Le nombre de retournements à effectuer est généralement compris entre 10 et 20 pour obtenir une agitation correcte. L'agitation peut s'effectuer par une succession de retournements partiels (figure 7) ou bien par une succession de retournements complets, l'amplitude du mouvement allant alors jusqu'à 180°.

Toutefois, l'invention ne se limite pas à un mode d'agitation par retournement et d'autres modes sont envisageables.

La figure 8 montre schématiquement un plateau horizontal 60 monté en rotation autour d'un axe horizontal 62 et agencé pour supporter un tube 24, l'axe longitudinal du tube étant disposé horizontalement et perpendiculaire à l'axe 62. Un tel plateau permet d'effectuer une succession de balancements du tube, ces balancements s'effectuent par exemple avec une amplitude de 180° par des rotations alternées entre - 90° et + 90°.

La figure 9 illustre un autre dispositif d'agitation 64 comprenant un support 66 entraîné en rotation autour d'un axe vertical 68 par un organe d'entraînement 70, tel qu'un moteur électrique du type pas à pas. Le support 66 est agencé pour recevoir un tube 24 dans une position verticale, avec le bouchon 26 dirigé vers le haut, l'axe longitudinal du tube coïncidant avec l'axe vertical 68 de rotation. Le dispositif 64 est agencé pour réaliser une séquence comprenant la rotation du tube de sang autour de l'axe vertical 68 avec un sens de rotation donné, un arrêt d'agitation, une rotation du tube de sang autour de l'axe vertical en sens inverse, et un nouvel arrêt de l'agitation et ainsi de suite.

Dans tous les cas, les moyens de commande (unité de commande 46) établissent une durée d'agitation qui détermine soit le nombre de retournements complets ou partiels du tube de sang (dispositif d'agitation 12), soit le nombre de balancements du tube de sang (dispositif d'agitation 61), soit le nombre de séquences de rotations et d'arrêts de rotation (dispositif d'agitation 64).

Le mode de fonctionnement de l'analyseur 10 est décrit ci-après.

Tout d'abord, le tube de sang 24 est placé dans le dispositif d'agitation soit manuellement, soit automatiquement comme déjà indiqué.

Le capteur 38 détecte la présence ou l'absence de bouchon 26. Le fonctionnement du dispositif d'agitation est conditionné par la détection d'un bouchon 26 par le capteur 38. Ensuite, l'agitation du tube de sang s'effectue dans des conditions contrôlées, et avec des paramètres définis, sous le contrôle de l'unité de commande 46.

Une fois l'agitation réalisée, le tube de sang se trouve à nouveau dans la position des figures 1 et 2, avec son bouchon dirigé vers le haut.

Le bloc de perçage 34 est alors placé automatiquement au-dessus du bouchon 26 et l'aiguille 36 est déplacée verticalement vers le bas pour percer le bouchon et permettre le prélèvement d'un échantillon de sang. Le prélèvement s'effectue en fonction du type d'analyse à réaliser. Il est souvent accompagné d'un moyen de ventilation du tube de sang, avant le prélèvement, de façon à prélever une quantité précise de sang.

Le bloc de perçage 34 est ensuite déplacé pour être amené au-dessus du bloc d'analyse et délivrer alors une quantité donnée d'échantillon, au travers de l'aiguille 36, dans un récipient de mesure 42. Les analyses sont ensuite effectuées par le bloc d'analyse 40, de manière classique, et les résultats sont fournis au module 44. A cette fin, l'analyseur de l'invention comprend des moyens de connexion interne ou externe (non représentés) permettant l'échange de données sécurisées.

Le tube 24 est avantageusement restitué à l'utilisateur lorsque les opérations d'agitation et de prélèvement sont terminées, et sans attendre que l'analyse en cours soit terminée.

Il est avantageux que le dispositif d'agitation et le bloc d'analyse soient agencés pour fonctionner de manière simultanée, de façon à agiter un tube de sang tandis qu'un tube précédemment agité est analysé.

Ainsi, un autre tube peut être mis en place pour être agité, tandis que l'analyse précédente est effectuée, ce qui permet de ne pas perdre de temps lorsque l'opérateur doit passer plusieurs tubes à la suite.

L'analyseur de l'invention permet aussi de régler la durée de l'agitation en fonction du tube à analyser, cette durée d'agitation déterminant le nombre d'agitations, comme déjà indiqué précédemment.

Les critères de choix de la durée d'agitation dépendent du type d'agitation retenu. On peut faire intervenir un facteur de forme du tube, plus ou moins favorable à un type d'agitation donné. On peut également retenir un facteur concernant le type de sang à analyser, si cela concerne un sang humain, un sang animal, ou un sang de contrôle ou de calibration. On peut retenir un facteur concernant le sang lui-même au moment de l'analyse. On peut sélectionner, par exemple, une durée plus longue pour un sang ayant été conservé pendant une longue période au réfrigérateur ou une durée plus courte pour un sang ayant été prélevé juste avant l'examen.

Dans l'analyseur de l'invention, le dispositif d'agitation peut être associé à des moyens de conservation d'échantillons destinés au contrôle qualité et à l'étalonnage de l'analyseur.

L'invention permet aussi de régler la vitesse des moyens d'agitation, par exemple la vitesse des retournements dans le cas d'une agitation par retournement complet ou partiel, ou la vitesse du balancement dans le cas d'une agitation par balancement.

Les paramètres de l'agitation, comme sa durée ou sa vitesse, peuvent être définis soit directement sur l'analyseur hématologique au moyen des touches 50 et de l'écran de contrôle 52, soit par l'intermédiaire d'une station de contrôle reliée à l'analyseur.

Les valeurs associées aux paramètres d'une agitation donnée peuvent être regroupées et enregistrées sous la forme de profils de manière à uniformiser simplement les conditions d'analyse.

Par exemple, on peut ainsi créer un profil pour les sangs retirés du réfrigérateur ou un autre pour les sangs immédiatement prélevés sur le patient.

La durée d'agitation peut être aussi réglée à une valeur nulle, ce qui permet de supprimer la séquence d'agitation.

Il est possible aussi de prévoir que l'analyseur ne lance pas d'analyse avant que la période d'agitation ne soit totalement terminée. Ceci prend toute son importance lorsque les analyses sont enchaînées, sans attendre la fin complète du cycle, dans la mesure où l'opérateur peut introduire le tube suivant à un moment quelconque.

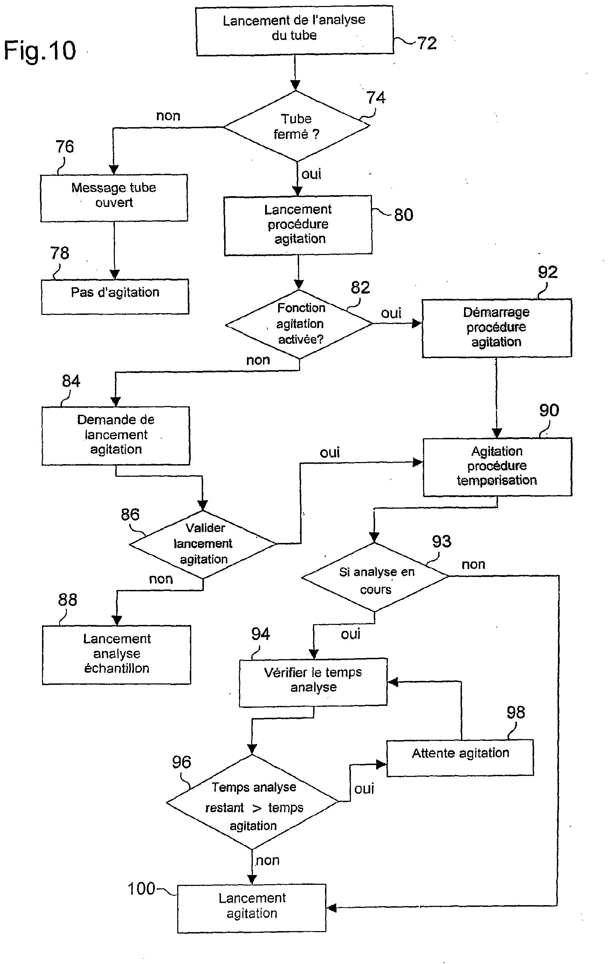

L'algorithme représenté à la figure 10 décrit le pilotage du dispositif d'agitation intégré à l'analyseur.

La procédure commence à l'étape 72 par le lancement de l'analyse du tube. A l'étape suivante 74 une comparaison est effectuée pour déterminer si le tube est ou non fermé. Cette information provient du capteur 38. Si le tube n'est pas fermé, un message tube ouvert est affiché à l'étape 76 et aucune agitation n'est réalisée comme mentionné à l'étape 78.

Dans le cas où le tube est fermé, l'étape 80 lance la procédure d'agitation.

A l'étape suivante 82, une comparaison est effectuée pour déterminer si la fonction agitation est activée.

Dans la négative, une demande de lancement d'agitation est faite à l'étape 84. Ensuite, à l'étape 86 une comparaison est effectuée pour valider le lancement de l'agitation.

Dans la négative, un lancement d'analyse d'échantillon est déclenché à l'étape 88. Dans l'affirmative, une étape 90 d'agitation et de procédure de temporisation est déclenchée.

Par ailleurs, si la comparaison de la fonction agitation activée de l'étape 82 est positive, le démarrage de la procédure d'agitation est effectuée à l'étape 92, et celle-ci aboutit à l'étape 90 déjà citée.

A la suite de l'étape 90, à l'étape 93, une comparaison est effectuée pour savoir si une analyse est en cours.

Dans l'affirmative, l'étape 94 vérifie le temps d'analyse.

A l'étape suivante 96, une comparaison est effectuée pour déterminer si le temps d'analyse restant est supérieur au temps d'agitation.

Dans l'affirmative, une étape 98 d'attente d'agitation est réalisée et cette étape retourne à l'étape 94.

Dans la négative, un lancement d'agitation est effectué à l'étape 100.

Ce lancement d'agitation peut être effectué aussi à partir de l'étape 93, dans le cas où la réponse est négative.

## Revendications

1. Analyseur hématologique sur sang total, pour l'analyse de sangs contenus dans des tubes, l'analyseur comprend:
- un dispositif d'agitation (12 ; 61 ; 64) agencé pour recevoir un tube de sang (24) et agiter ce tube de sang selon un mode d'agitation choisi ;
- des moyens de commande (44, 46) reliés au dispositif d'agitation (12 ; 61 ; 64) pour agiter le tube de sang (24) dans des conditions contrôlées selon des paramètres définis ; et
- des moyens de prélèvement (32) agencés pour prélever un échantillon de sang dans le tube de sang (24) préalablement agité par le dispositif d'agitation et transférer l'échantillon de sang vers un bloc d'analyse (40) **caractérisé en ce que** les tubes sont amenés et traités dans un mode tube par tube.

2. Analyseur hématologique selon la revendication 1, **caractérisé en ce qu'**il comprend des moyens de déplacement (54) agencés pour transférer un tube de sang (24) vers le dispositif d'agitation (12 ; 61 ; 64).

3. Analyseur hématologique selon l'une des revendications 1 et 2, **caractérisé en ce que** le dispositif d'agitation (12 ; 61 ; 64) comprend un organe de réception (22) pour recevoir un tube de sang (24) soit directement, soit par l'intermédiaire d'un support (58).

4. Analyseur hématologique selon la revendication 3, **caractérisé en ce que** l'organe de réception comporte soit une seule empreinte (22) adaptée à un tube de sang d'un modèle défini, soit plusieurs empreintes (22-1, 22-2, 22-3) adaptées respectivement à des tubes de sang de modèles différents.

5. Analyseur hématologique selon l'une des revendications 1 à 4, **caractérisé en ce que** les moyens de commande (46) comprennent des moyens de programmation (47) pour programmer la durée de l'agitation du dispositif d'agitation.

6. Analyseur hématologique selon l'une des revendications 1 à 5, **caractérisé en ce que** le dispositif d'agitation (12) comprend des moyens d'agitation par une succession de retournements complets du tube de sang.

7. Analyseur hématologique selon l'une des revendications 1 à 5, **caractérisé en ce que** le dispositif d'agitation (12) comprend des moyens d'agitation par une succession de retournements partiels du tube de sang.

8. Analyseur hématologique selon l'une des revendications 1 à 5, **caractérisé en ce que** le dispositif d'agitation (61) comprend des moyens d'agitation par une succession de balancements du tube de sang dans un plan vertical.

9. Analyseur hématologique selon l'une des revendications 1 à 5, **caractérisé en ce que** le dispositif d'agitation (64) comprend des moyens d'agitation par une séquence comprenant la rotation du tube de sang selon un axe vertical (68) avec un sens de rotation donné, un arrêt de l'agitation, une rotation du tube de sang selon l'axe vertical (68) en sens inverse, et un nouvel arrêt de l'agitation.

10. Analyseur hématologique selon la revendication 6, **caractérisé en ce que** les moyens de commande (46) établissent une durée d'agitation qui détermine le nombre de retournements complets du tube de sang.

11. Analyseur hématologique selon la revendication 8, **caractérisé en ce que** les moyens de commande (46) établissent une durée d'agitation qui détermine le nombre de balancements du tube de sang.

12. Analyseur hématologique selon la revendication 9, **caractérisé en ce que** les moyens de commande (46) établissent une durée d'agitation qui détermine le nombre de séquences de rotations et d'arrêts de rotation.

13. Analyseur hématologique selon l'une des revendications 1 à 12, **caractérisé en ce que** les paramètres d'agitation des moyens de commande (46) sont paramétrables soit directement sur l'analyseur hématologique, soit par l'intermédiaire d'une station de contrôle indépendante.

14. Analyseur hématologique selon la revendication 13, **caractérisé en ce que** les paramètres d'agitation sont regroupés en profils d'utilisation.

15. Analyseur hématologique selon l'une des revendications 1 à 14, **caractérisé en ce que** le dispositif d'agitation (12 ; 61 ; 64) et le bloc d'analyse (40) sont agencés pour fonctionner de manière simultanée, ce qui permet d'agiter un tube de sang par les moyens d'agitation, tandis qu'un tube précédemment agité est analysé par le bloc d'analyse.

16. Analyseur hématologique selon l'une des revendications 1 à 15, **caractérisé en ce que** les moyens de commande (46) établissent une durée d'agitation qui peut être nulle, ce qui permet de ne pas effectuer une agitation.

17. Analyseur hématologique selon l'une des revendications 1 à 16, dans lequel le tube de sang (24) est fermé par un bouchon (26), **caractérisé en ce qu'**un capteur optique (38) est placé à l'intérieur de l'analyseur hématologique pour détecter la présence ou l'absence d'un bouchon sur le tube de sang.

18. Analyseur hématologique selon l'une des revendications 1 à 17, dans lequel le tube de sang (24) est fermé par un bouchon (26), **caractérisé en ce que** les moyens de prélèvement (32) comprennent un organe de perçage (34) du bouchon, qui est situé en surplomb du tube à percer.

19. Analyseur hématologique selon l'une des revendications 1 à 17, dans lequel le tube de sang (24) est fermé par un bouchon (26) **caractérisé en ce que** les moyens de prélèvement (32) comprennent un organe de perçage (34) du bouchon qui est situé en dessous du tube à percer.

20. Analyseur hématologique selon l'une des revendications 1 à 19, **caractérisé en ce qu'**il comprend des moyens de connexion interne ou externe permettant l'échange de données sécurisées.

## Patentansprüche

1. Hämatologisches Analysegerät für Vollblut, für die Analyse von in Röhrchen enthaltenem Blut, wobei das Analysegerät Folgendes umfasst:
- eine Rührvorrichtung (12; 61; 64), die so angeordnet ist, dass sie ein Blutröhrchen (24) aufnimmt und das Blutröhrchen in einem ausgewählten Rührmodus rührt;
- Steuermittel (44, 46), die mit der Rührvorrichtung (12; 61; 64) verbunden sind, um das Blutröhrchen (24) unter kontrollierten Bedingungen nach definierten Parametern zu rühren; und
- Probenahmemittel (32), die zur Entnahme einer Blutprobe aus dem zuvor von der Rührvorrichtung gerührten Blutröhrchen (24) und zur Übertragung der Blutprobe auf einen Analyseblock (40) angeordnet sind, **dadurch gekennzeichnet, dass** die Röhrchen in einem Röhrchen-für-Röhrchen-Modus zugeführt und verarbeitet werden.

2. Hämatologisches Analysegerät nach Anspruch 1, **dadurch gekennzeichnet, dass** es Bewegungsmittel (54) umfasst, die so angeordnet sind, dass sie ein Blutröhrchen (24) zu der Rührvorrichtung (12; 61; 64) übertragen.

3. Hämatologisches Analysegerät nach einem der Ansprüche 1 und 2, **dadurch gekennzeichnet, dass** die Rührvorrichtung (12; 61; 64) ein Aufnahmeelement (22) zur Aufnahme eines Blutröhrchens (24) entweder direkt oder mittels eines Trägers (58) umfasst.

4. Hämatologisches Analysegerät nach Anspruch 3, **dadurch gekennzeichnet, dass** das Aufnahmeelement entweder eine einzelne Prägung (22) umfasst, die an ein Blutröhrchen eines definierten Modells angepasst ist, oder mehrere Prägungen (22-1, 22-2, 22-3), die jeweils an Blutröhrchen verschiedener Modelle angepasst sind.

5. Hämatologisches Analysegerät nach einem der Ansprüche 1 bis 4, **dadurch gekennzeichnet, dass** die Steuermittel (46) Programmiermittel (47) zur Programmierung der Rührdauer der Rührvorrichtung umfassen.

6. Hämatologisches Analysegerät nach einem der Ansprüche 1 bis 5, **dadurch gekennzeichnet, dass** die Rührvorrichtung (12) Rührmittel durch aufeinanderfolgende vollständige Umdrehungen des Blutröhrchens umfasst.

7. Hämatologisches Analysegerät nach einem der Ansprüche 1 bis 5, **dadurch gekennzeichnet, dass** die Rührvorrichtung (12) Rührmittel durch aufeinanderfolgende partielle Umdrehungen des Blutröhrchens umfasst.

8. Hämatologisches Analysegerät nach einem der Ansprüche 1 bis 5, **dadurch gekennzeichnet, dass** die Rührvorrichtung (61) Rührmittel zum Rühren durch aufeinanderfolgende Schwingungen des Blutröhrchens in einer vertikalen Ebene umfasst.

9. Hämatologisches Analysegerät nach einem der Ansprüche 1 bis 5, **dadurch gekennzeichnet, dass** die Rührvorrichtung (64) Rührmittel durch eine Sequenz umfasst, die das Drehen des Blutröhrchens entlang einer vertikalen Achse (68) mit einer gegebenen Drehrichtung, ein Stoppen des Rührens, ein Drehen des Blutröhrchens entlang der vertikalen Achse (68) in der entgegengesetzten Richtung und ein erneutes Stoppen des Rührens umfasst.

10. Hämatologisches Analysegerät nach Anspruch 6, **dadurch gekennzeichnet, dass** die Steuermittel (46) eine Rührdauer festlegen, welche die Anzahl der vollständigen Umdrehungen des Blutröhrchens bestimmt.

11. Hämatologisches Analysegerät nach Anspruch 8, **dadurch gekennzeichnet, dass** die Steuermittel (46) eine Rührdauer festlegen, welche die Anzahl der Schwingungen des Blutröhrchens bestimmt.

12. Hämatologisches Analysegerät nach Anspruch 9, **dadurch gekennzeichnet, dass** die Steuermittel (46) eine Rührdauer festlegen, welche die Anzahl der die Anzahl der Drehsequenzen und der Drehstopps bestimmt.

13. Hämatologisches Analysegerät nach einem der Ansprüche 1 bis 12, **dadurch gekennzeichnet, dass** die Rührparameter der Steuermittel (46) entweder direkt am hämatologischen Analysegerät oder mittels einer eigenständigen Kontrollstation parametrierbar sind.

14. Hämatologisches Analysegerät nach Anspruch 13, **dadurch gekennzeichnet, dass** die Rührparameter in Nutzungsprofile gruppiert sind.

15. Hämatologisches Analysegerät nach einem der Ansprüche 1 bis 14, **dadurch gekennzeichnet, dass** die Rührvorrichtung (12; 61; 64) und der Analyseblock (40) so angeordnet sind, dass sie gleichzeitig arbeiten, wodurch das Rühren eines Blutröhrchens durch die Rührmittel ermöglicht wird, während ein zuvor gerührtes Röhrchen durch den Analyseblock analysiert wird.

16. Hämatologisches Analysegerät nach einem der Ansprüche 1 bis 15, **dadurch gekennzeichnet, dass** die Steuermittel (46) eine Rührdauer festlegen, die Null sein kann, wodurch kein Rührvorgang erfolgt.

17. Hämatologisches Analysegerät nach einem der Ansprüche 1 bis 16, wobei das Blutröhrchen (24) mit einem Stopfen (26) verschlossen ist, **dadurch gekennzeichnet, dass** ein optischer Sensor (38) im Inneren des hämatologischen Analysegeräts angebracht ist, um das Vorhandensein oder Fehlen eines Stopfens auf dem Blutröhrchen zu erkennen.

18. Hämatologisches Analysegerät nach einem der Ansprüche 1 bis 17, wobei das Blutröhrchen (24) mit einem Stopfen (26) verschlossen ist, **dadurch gekennzeichnet, dass** die Probenahmemittel (32) ein Durchstechelement (34) des Stopfens umfassen, das zum durchzustechenden Röhrchen überhängend angeordnet ist.

19. Hämatologisches Analysegerät nach einem der Ansprüche 1 bis 17, wobei das Blutröhrchen (24) mit einem Stopfen (26) verschlossen ist, **dadurch gekennzeichnet, dass** die Probenahmemittel (32) ein Durchstechelement (34) des Stopfens umfassen, das unterhalb des durchzustechenden Röhrchens angeordnet ist.

20. Hämatologisches Analysegerät nach einem der Ansprüche 1 bis 19, **gekennzeichnet dadurch, dass** es Mittel zur internen oder externen Verbindung umfasst, die den Austausch von gesicherten Daten ermöglichen.

## Claims

1. Blood analyser of whole blood for the analysis of bloods contained in tubes, the analyser comprises:
- a stirring device (12; 61; 64) arranged to receive one tube of blood (24) and to stir this tube of blood according to a selected manner of stirring;
- control means (44, 46) connected to the stirring device (12; 61; 64) in order to stir the tube of blood (24) in conditions controlled according to defined parameters; and
- sampling means (32) arranged to remove a sample of blood from the tube of blood (24) previously stirred by the stirring device and to transfer the sample of blood to an analysis block (40)
**characterised in that** the tubes are supplied and processed in a tube-by-tube manner.

2. Blood analyser according to claim 1, **characterised in that** it comprises displacement means (54) arranged to transfer a tube of blood (24) to the stirring device (12; 61; 64)

3. Blood analyser according to either of claims 1 or 2, **characterised in that** the stirring device (12; 61; 64) comprises a receiving element (22) for receiving a tube of blood (24) either directly or via a support (58).

4. Blood analyser according to claim 3, **characterised in that** the receiving element comprises either a single recess (22) adapted to one tube of blood of a specified shape, or plural recesses (22-1, 22-2, 22-3) adapted respectively to tubes of blood of different shapes.

5. Blood analyser according to one of claims 1 to 4, **characterised in that** the control means (46) comprise programming means (47) for programming the duration of stirring of the stirring device.

6. Blood analyser according to one of claims 1 to 5, **characterised in that** the stirring device (12) comprises means of stirring by a succession of full inversions of the tube of blood.

7. Blood analyser according to one of claims 1 to 5, **characterised in that** the stirring device (61) comprises means for stirring by a succession of partial inversions of the tube of blood.

8. Blood analyser according to one of claims 1 to 5, **characterised in that** the stirring device (61) comprises means of stirring by a succession of rocking motions of the tube of blood in a vertical plane.

9. Blood analyser according to one of claims 1 to 5, **characterised in that** the stirring device (64) comprises means of stirring by a sequence comprising rotation of the tube of blood about a vertical axis (68) with a specified direction of rotation, halting of stirring, rotation of the tube of blood about the vertical axis (68) in the opposite direction, and halting once more of stirring.

10. Blood analyser according to claim 6, **characterised in that** the control means (46) set a duration of stirring which determines the number of full inversions of the tube of blood.

11. Blood analyser according to claim 8, **characterised in that** the control means (46) set a duration of stirring which determines the number of rocking motions of the tube of blood.

12. Blood analyser according to claim 9, **characterised in that** the control means (46) set a duration of stirring which determines the number of sequences of rotations and halts in rotation.

13. Blood analyser according to one of claims 1 to 12, **characterised in that** the stirring parameters of the control means (46) may be set either directly on the blood analyser, or via an independent control station.

14. Blood analyser according to claim 13, **characterised in that** the stirring parameters are assembled into profiles of use.

15. Blood analyser according to one of claims 1 to 14, **characterised in that** the stirring device (12; 61; 64) and the analysis block (40) are arranged to operate simultaneously, which makes it possible to stir a tube of blood by the stirring means white a previously stirred tube is being analysed by the analysis block.

16. Blood analyser according to one of claims 1 to 15, **characterised in that** the control means (46) set a duration of stirring which may be zero, which makes it possible not to carry out stirring.

17. Blood analyser according to one of claims 1 to 16, wherein the tube of blood (24) is sealed by a stopper (26), **characterised in that** an optical sensor (38) is placed inside the blood analyser in order to detect the presence or absence of a stopper on the tube of blood.

18. Blood analyser according to one of claims 1 to 17, wherein the tube of blood (24) is sealed by a stopper (26), **characterised in that** the sampling means (32) comprise an element (34) for piercing the stopper, which piercing element is located directly above the tube to be pierced.

19. Blood analyser according to one of claims 1 to 17, wherein the tube of blood (24) is sealed by a stopper (26), **characterised in that** the sampling means (32) comprise an element (34) for piercing the stopper, which piercing element is located directly below the tube to be pierced.

20. Blood analyser according to one of claims 1 to 19, **characterised in that** it comprises internal or external connecting means for the exchange of securitised data.
